(19) Europäisches Patentamt
European Patent Office
Office européen des brevets

(11) Veröffentlichungsnummer: **0 003 796**
**B1**

(12) # EUROPÄISCHE PATENTSCHRIFT

(21) Anmeldenummer: **79100424.5**

(22) Anmeldetag: **13.02.79**

(51) Int. Cl.$^3$: **C 07 D 233/58,**
A 61 K 31/415

(54) **Substituierte Diphenyl-imidazolyl-methane, Verfahren zu ihrer Herstellung sowie sie enthaltende Arzneimittel**

(30) Priorität: **24.02.78 DE 2808086**

(43) Veröffentlichungstag der Anmeldung:
**05.09.79 Patentblatt 79/18**

(45) Bekanntmachung des Hinweises auf die Patenterteilung:
**15.10.80 Patentblatt 80/21**

(84) Benannte Vertragsstaaten:
**BE CH DE FR GB IT LU NL SE**

(56) Entgegenhaltungen:
**DE - A - 2 130 673**
**DE - A - 2 418 502**
**DE - A - 2 461 406**
**DE - A - 2 510 130**

(73) Patentinhaber: **BAYER AG**
**Zentralbereich Patente, Marken und Lizenzen**
**D - 5090 Leverkusen 1**
**Bayerwerk (DE)**

(72) Erfinder: **Regel, Erik, Ing. grad.**
**Bergerheide 72a**
**D - 5600 Wuppertal 1 (DE)**
**Büchel, Karl Heinz, Prof., Dr.**
**Bergerheide 62**
**D - 5600 Wuppertal 1 (DE)**
**Draber, Wilfried, Dr.**
**In den Birken 81**
**D - 5600 Wuppertal 1 (DE)**
**Plempel, Manfred, Dr.**
**Pahlkestrasse 5**
**D - 5600 Wuppertal 1 (DE)**
**Haller, Ingo, Dr.**
**Viktoriastrasse 99**
**D - 5600 Wuppertal 1 (DE)**

**EP 0 003 796 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Substituierte Diphenyl-imidazolyl-methane, Verfahren zu ihrer Herstellung sowie sie enthaltende Arzneimittel

Die Erfindung betrifft neue substituierte Diphenyl-imidazolyl-methane, ein Verfahren zu ihrer Herstellung sowie sie enthaltende Arzneimittel, insbesondere als Antimykotika.

Es ist bereits bekannt geworden, daß Biphenyl-imidazolyl-methane-Derivate gute antimykotische Wirkung aufweisen (vgl. DT—OS 2 418 502 und DT—OS 2 461 406 [Le A 16 148]. Jedoch ist deren Wirkung nicht bei allen Pilzspezies immer ganz befriedigend. Weiterhin ist bereits bekannt, daß 2-Chlorphenyl-imidazol-1-yl-naphth-1-yl-methan antimykotische Eigenschaften besitzt (vgl. J. Pharm. Sci. *62,* 773 bis 778 (1973)). Auch deren Wirkung ist jedoch, insbesondere gegen Sproß- und Schimmelpilze, nicht immer ganz befriedigend.

Es wurden nun als neue Verbindungen die substituierten Diphenyl-imidazolyl-methane der Formel

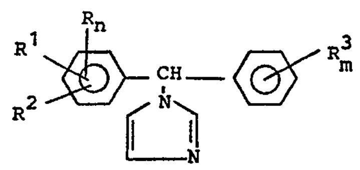

(I)

in welcher

R für Halogen, geradkettiges oder verzweigtes Alkyl oder Alkoxy mit jeweils 1 bis 4 Kohlenstoffatomen sowie für Halogenalkyl mit 1 bis 2 Kohlenstoffatomen und bis zu 3 Halogenatomen steht,

$R^1$ für gegebenenfalls einfach oder mehrfach durch Halogen oder Alkyl mit 1 bis 4 Kohlenstoffatomen substituiertes Cycloalkxl mit 3 bis 7 Kohlenstoffatomen steht,

$R^2$ für Wasserstoff steht, oder

$R^1$ und $R^2$ gemeinsam in ortho-Stellung zueinander für eine gegebenenfalls einfach oder mehrfach durch Halogen oder Alkyl mit 1 bis 4 Kohlenstoffatomen substituierte Methylenbrücke mit 3 bis 5 Methylengruppen stehen,

$R^3$ für Halogen, geradkettiges oder verzweigtes Alkyl mit 1 bis 4 Kohlenstoffatomen, Halogenalkyl mit 1 bis 2 Kohlenstoffatomen und bis zu 3 Halogenatomen, Alkoxy, Alkylthio und Alkylsulfonyl mit jeweils 1 bis 2 Kohlenstoffatomen, Amino, Nitro oder Cyano, und

m und n für ganze Zahlen von 0 bis 3 stehen,

und deren physiologisch verträglichen Säureadditionssalze, gefunden. Sie weisen starke antimykotische Eigenschaften auf.

Weiterhin wurde gefunden, daß man die neuen Diphenylimidazolyl-methane der Formel (I) erhält, wenn man Diphenyl-halogen-methane der Formel

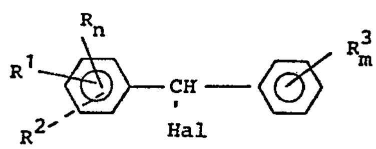

(II)

in welcher

R, $R^1$, $R^2$, $R^3$, m und n die oben angegebene Bedeutung haben und

Hal für Halogen steht,

mit Imidazol, gegebenenfalls in Gegenwart eines Säurebindemittels und gegebenenfalls in Gegenwart eines Verdünnungsmittels, umsetzt.

In manchen Fällen erweist es sich als vorteilhaft, anstelle des Imidazols das Silber- oder Alkalisalz, wie das Natrium- oder Kaliumsalz, einzusetzen.

Weiterhin können die erfindungsgemäß erhältlichen Diphenyl-imidazolyl-methane durch Umsetzen mit Säuren in die Salze überführt werden.

Überraschenderweise zeigen die erfindungsgemäßen Diphenyl-imidazolyl-methane eine bessere und breitere antimykotische, therapeutisch nutzbare Wirksamkeit als die aus dem Stand der Technik bekannten Biphenyl-imidazolyl-methan-Derivate und als das bekannte 2-Chlorphenyl-imidazol-1-yl-naphth-1-yl-methan, welches chemisch und wirkungsmäßig naheliegendste Verbindungen sind. Die erfindungsgemäßen Wirkstoffe stellen somit eine wertvolle Bereicherung der Pharmazie dar.

Verwendet man 2-Chlorphenyl-indan-5-yl-chlormethan und Imidazol als Ausgangsstoffe, so kann der Reaktionsablauf durch das folgende Formelschema wiedergegeben werden:

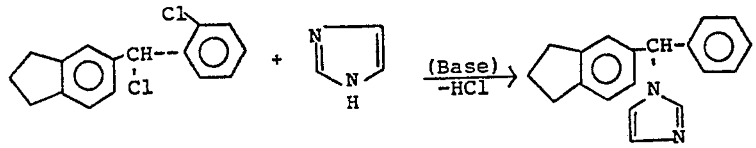

Die als Ausgangsstoffe zu verwendenden Diphenyl-halogen-methane der Formel (II) sind noch nicht bekannt. Sie können jedoch nach üblichen Methoden erhalten werden, indem man Carbinole der Formel

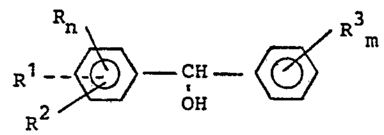

(III)

in welcher

R, $R^1$, $R^2$, $R^3$, m und n die oben angegebene Bedeutung haben,

in bekannter Weise halogeniert, wie z.B. mit Chlorwasserstoff (vgl. hierzu J. Org. Chem. *36,* (18), 2724 (1971)) oder mit Thionylchlorid (vgl. hierzu Izv. Akad. SSSR *10,* 1804 (1962) sowie die Herstellungsbeispiele).

Die Carbinole der Formel (III) sind ebenfalls noch nicht bekannt, können aber nach üblichen Methoden hergestellt werden. Man erhält sie z.B. durch Reduktion der nach einer Friedel-Crafts-Reaktion erhaltenen Ketone mit Aluminiumisopropylat (vgl. hierzu Izv. Akad. SSSR *10,* 1804 (1962) und Ž. obšč. Chim. *34,* (3), 977 (1964) und Ž. org. Chim. *2,* (7), 1288 (1966)). Die Reduktion kann aber auch mit jedem anderen Reduktionsmittel, wie z.B. Natriumborhydrid, durchgeführt werden (vgl. hierzu die Herstellungsbeispiele).

Für die erfindungsgemäße Umsetzung kommen als Verdünnungsmittel vorzugsweise inerte organische Lösungsmittel in Frage. Hierzu gehören vorzugsweise Ketone, wie Diäthylketon, insbesondere Aceton und Methyläthylketon; Nitrile, wie Propionitril, insbesondere Acetonitril; Alkohole, wie Aethanol oder Isopropanol; Aether, wie Tetrahydrofuran oder Dioxan; aromatische Kohlenwasserstoff, wie Benzol, Toluol und Dichlorbenzol; Formamide, wie insbesondere Dimethylformamid; und halogenierte Kohlenwasserstoffe, wie Methylenchlorid, Tetrachlorkohlenstoff oder Chloroform.

Wird die erfindungsgemäße Umsetzung in Gegenwart eines Säurebinders vorgenommen, so kann man alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triäthylamin, N,N-Dimethylcyclohexylamin, Dicyclohexylmethylamin, N,N-Dimethylbenzylamin, weiterhin, Pyridin und Diazabicyclooctan. Vorzugsweise verwendet man einen Überschuß an Imidazol.

Die Reaktionstemperaturen können in einem größern Bereich variiert werden. Im allgemeinen arbeitet man zwischen etwa 30 bis etwa 200°C, vorzugsweise bei der Siedetemperatur des Lösungsmittels.

Bei der Durchführung des erfindungsgemäßen Verfahrens setzt man auf 1 Mol der Verbindungen der Formel (II) vorzugsweise 1 bis 2,5 Mol Imidazol und 1 bis 2,5 Mol Säurebinder ein. Zur Isolierung der Verbindungen der Formel (I) wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird gegebenenfalls durch Destillation, Umkristallisation oder chromatographisch gereinigt.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z.B. die Chlorwasserstoff-säure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure sowie Sulfonsäure, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Als Beispiele für besonders wirksame Vertreter der erfindungsgemäßen Wirkstoffe seien außer den Herstellungsbeispielen und den Beispielen der Tabelle 1 genannt:

4-Cyclopropylphenyl-2-fluorphenyl-imidazol-1-yl-methan
4-Cyclohexylphenyl-4-chlorphenyl-imidazol-1-yl-methan
4-Cyclohexylphenyl-4-fluorphenyl-imidazol-1-yl-methan
4-Cyclohexylphenyl-2-fluorphenyl-imidazol-1-yl-methan
4-Cyclohexylphenyl-2,4-dichlorphenyl-imidazol-1-yl-methan
4-Chlor-3-cyclohexylphenyl-2-chlorphenyl-imidazol-1-yl-methan
4-Chlor-3-cyclohexylphenyl-4-chlorphenyl-imidazol-1-yl-methan
4-Chlor-3-cyclohexylphenyl-2-fluorphenyl-imidazol-1-yl-methan
4-Chlor-3-cyclohexylphenyl-4-fluorphenyl-imidazol-1-yl-methan
4-Chlor-3-cyclohexylphenyl-2,4-dichlorphenyl-imidazol-1-yl-methan
4-(3-Bromcyclohexyl)-phenyl-2-chlorphenyl-imidazol-1-yl-methan
4-(3-Bromcyclohexyl)-phenyl-4-chlorphenyl-imidazol-1-yl-methan
4-(3-Bromcyclohexyl)-phenyl-2-fluorphenyl-imidazol-1-yl-methan
4-(3-Bromcyclohexyl)-phenyl-4-fluorphenyl-imidazol-1-yl-methan
4-(3-Bromcyclohexyl)-phenyl-2,4-dichlorphenyl-imidazol-1-yl-methan
4-Cyclopentyl-phenyl-imidazol-1-yl-methan
4-Cyclopentyl-2-chlorphenyl-imidazol-1-yl-methan
4-Cyclopentyl-4-chlorphenyl-imidazol-1-yl-methan
4-Cyclopentyl-2-fluorphenyl-imidazol-1-yl-methan
4-Cyclopentyl-4-fluorphenyl-imidazol-1-yl-methan
4-Cyclopentyl-2,4-dichlorphenyl-imidazol-1-yl-methan
4-Cyclopentyl-2-methylphenyl-phenyl-imidazol-1-yl-methan
4-Cyclopentyl-2-methylphenyl-2-chlorphenyl-imidazol-1-yl-methan
4-Cyclopentyl-2-methylphenyl-4-chlorphenyl-imidazol-1-yl-methan
4-Cyclopentyl-2-methylphenyl-2-fluorphenyl-imidazol-1-yl-methan
4-Cyclopentyl-2-methylphenyl-4-fluorphenyl-imidazol-1-yl-methan
4-Cyclopentyl-2-methylphenyl-2,4-dichlorphenyl-imidazol-1-yl-methan
4-(1-Methylcyclohexyl)-phenyl-imidazol-1-yl-methan
4-(1-Methylcyclohexyl)-phenyl-2-chlorphenyl-imidazol-1-yl-methan
4-(1-Methylcyclohexyl)-phenyl-4-chlorphenyl-imidazol-1-yl-methan
4-(1-Methylcyclohexyl)-phenyl-2-fluorphenyl-imidazol-1-yl-methan
4-(1-Methylcyclohexyl)-phenyl-4-fluorphenyl-imidazol-1-yl-methan
4-(1-Methylcyclohexyl)-phenyl-2,4-dichlorphenyl-imidazol-1-yl-methan
4-Cycloheptyl-phenyl-imidazol-1-yl-methan
4-Cycloheptyl-2-chlorphenyl-imidazol-1-yl-methan
4-Cycloheptyl-4-chlorphenyl-imidazol-1-yl-methan
4-Cycloheptyl-2-fluorphenyl-imidazol-1-yl-methan
4-Cycloheptyl-4-fluorphenyl-imidazol-1-yl-methan
4-Cycloheptyl-2,4-dichlorphenyl-imidazol-1-yl-methan
6-Chlor-1,2,3,4-tetrahydro-naphth-7-yl-phenyl-imidazol-1-yl-methan
6-Chlor-1,2,3,4-tetrahydro-naphth-7-yl-2-chlorphenyl-imidazol-1-yl-methan
6-Chlor-1,2,3,4-tetrahydro-naphth-7-yl-2-fluorphenyl-imidazol-1-yl-methan
5-Chlor-1,2,3,4-tetrahydro-naphth-8-yl-phenyl-imidazol-1-yl-methan
5-Chlor-1,2,3,4-tetrahydro-naphth-8-yl-2-chlorphenyl-imidazol-1-yl-methan
5-Chlor-1,2,3,4-tetrahydro-naphth-8-yl-2-bromphenyl-imidazol-1-yl-methan
2-Chlor-1,2,3,4-tetrahydro-naphth-6-yl-phenyl-imidazol-1-yl-methan
2-Chlor-1,2,3,4-tetrahydro-naphth-6-yl-2-chlorphenyl-imidazol-1-yl-methan
2-Chlor-1,2,3,4-tetrahydro-naphth-6-yl-2-fluorphenyl-imidazol-1-yl-methan
1,2,3,4-Tetrachlor-1,2,3,4-tetrahydro-naphth-6-yl-phenyl-imidazol-1-yl-methan
1,2,3,4-Tetrachlor-1,2,3,4-tetrahydro-naphth-6-yl-2-chlorphenyl-imidazol-1-yl-methan
1,2,3,4-Tetrachlor-1,2,3,4-tetrahydro-naphth-6-yl-2-fluorphenyl-imidazol-1-yl-methan
1,2,3,4-Tetrachlor-1,2,3,4-tetrahydro-naphth-6-yl-4-chlorphenyl-imidazol-1-yl-methan
5-Methyl-1,2,3,4-tetrahydro-naphth-7-yl-phenyl-imidazol-1-yl-methan
5-Methyl-1,2,3,4-tetrahydro-naphth-7-yl-2-chlorphenyl-imidazol-1-yl-methan
5-Methyl-1,2,3,4-tetrahydro-naphth-7-yl-2-fluorphenyl-imidazol-1-yl-methan
5-Methyl-1,2,3,4-tetrahydro-naphth-7-yl-2-methylphenyl-imidazol-1-yl-methan
5-Methyl-1,2,3,4-tetrahydro-naphth-7-yl-2,6-difluorphenyl-imidazol-1-yl-methan
3,4-Pentamethylenphenyl-phenyl-imidazol-1-yl-methan
3,4-Pentamethylenphenyl-2-chlorphenyl-imidazol-1-yl-methan
3,4-Pentamethylenphenyl-2-fluorphenyl-imidazol-1-yl-methan
Indan-4-yl-phenyl-imidazol-1-yl-methan
Indan-4-yl-2-chlorphenyl-imidazol-1-yl-methan
Indan-4-yl-2-fluorphenyl-imidazol-1-yl-methan
Indan-4-yl-2-bromphenyl-imidazol-1-yl-methan
Indan-4-yl-2-methylphenyl-imidazol-1-yl-methan

Indan-4-yl-2,3-dimethylphenyl-imidazol-1-yl-methan
Indan-5-yl-2-bromphenyl-imidazol-1-yl-methan
3-Chlor-indan-5-yl-phenyl-imidazol-1-yl-methan
3-Chlor-indan-5-yl-2-chlorphenyl-imidazol-1-yl-methan
3-Chlor-indan-5-yl-4-chlorphenyl-imidazol-1-yl-methan
3-Chlor-indan-5-yl-2-fluorphenyl-imidazol-1-yl-methan
6-Chlor-indan-5-yl-phenyl-imidazol-1-yl-methan
6-Chlor-indan-5-yl-2-chlorphenyl-imidazol-1-yl-methan
6-Chlor-indan-5-yl-2-fluorphenyl-imidazol-1-yl-methan
3-Methyl-indan-5-yl-phenyl-imidazol-1-yl-methan
3-Methyl-indan-5-yl-2-chlorphenyl-imidazol-1-yl-methan
3-Methyl-indan-5-yl-2-fluorphenyl-imidazol-1-yl-methan

Die erfindungsgemäß verwendbaren Verbindungen der Formel (I) und ihre Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasiche Pilze, z.B. gegen Candida-Arten, wie Candida albicans, Epidermophyton-Arten, wie Epidermophyton floccosum Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporon-Arten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und systemmykosen, insbesondere solche, die durch die obengenannten Erreger hervorgerufen werden.

Zur vorliegenden Erfindung gehören pharmazeutische Zubereitungen, die neben nichttoxischen, inerten pharmzeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant,

Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Aethylalkohol, Isoprooylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Aethylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Sacharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös, appliziert werden.

Im allgemeinen hat es sich sowohl in der Human- als auch in der Veterinärmedizin als vorteilhaft erwiesen, den oder die erfindungsgemäßen Wirkstoff in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Beispiel A

### *Antimykotische in vitro-Wirksamkeit*

Versuchsbeschreibung:

Die in vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze: Sabouraud's milieu d'épreuve

b) für Hefen: Fleischextrakt-Traubenzucker-Bouillon

Die Bebrütungstemperatur betrug 28°C, die Bebrütungsdauer lag bei 24 bis 96 Stunden.

Bei diesen in vitro-Prüfungen zeigen z.B. die erfindungsgemäßen Verbindungen der Beispiele 1, 2, 3, 4, 5, 6, 7, 8, 9 und 10 gute antimykotische Eigenschaften, die denjenigen der aus dem Stand der Technik bekannten Verbindungen überlegen sind.

## Beispiel B

### *Antimykotische in vivo-Wirksamkeit (lokal) am Modell der experimentellen Meerschweinchen-Trichophytie*

Versuchsbeschreibung:

Weiße Meerschweinchen der Rasse Pirbright-white wurden auf dem geschorenen, nicht skarifizierten Rücken mit einer Mikro- und Makrokonidien-Suspension von Trichophyton mentagrophytes infiziert. Bei unbehandelten Tieren entwickelt sich innerhalbt 12 Tagen p.i. das typi-

sche Bild einer Dermantophytose mit Rötung, Schuppung und Haarausfall bis zum totalen Integument-Defekt an der Infektionsstelle. Die infizierten Tiere wurden — beginnend mit dem 3. Tag p.i. — 1 mal täglich mit 1%igen Polyäthylenglykol-Lösungen der Vergleichs- und erfindungsgemäßen Präparate lokal behandelt.

Am 14. Tag p.i. zeigten die unbehandelten Kontrolltiere sowie die mit den Vergleichswirkstoffen behandelten das typische Bild einer Dermatophytose, während die Prüfpräparate den Ablauf der Infektion gehemmt hatten.

Die so durchgeführten Versuche zeigen z.B. gute antimykotische in vivo-Wirksamkeit der erfindungsgemäßen Verbindungen der Beispiele 4, 6, 7, 9 und 10.

*Herstellungsbeispiele*

Beispiel 1

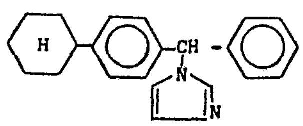

Zu einer Suspension von 44,3 g (0,65 Mol) Imidazol in 100 ml 1,2-Dichlorbenzol werden bei 170°C 37 g (0,13 Mol) 4-Cyclohexylbenzhydrylchlorid portionsweise eingetragen. Nach einstündigem Rühren bei 170°C wird das Reaktionsgemisch am Rotationsverdampfer bei 80 bis 90°C eingedampft und der Rückstand in Methylenchlorid gelöst. Nach mehrmaligem Waschen der organischen Phase mit Wasser wird die Methylenchlorid-Lösung über Natriumsulfat getrocknet und eingedampft. Das verbleibende Oel wird mit Petroläther extrahiert, die Petroläther-Lösung eingedampft und das resultierende Oel chromatographisch gereinigt (Absorbtionsmittel: Kieselgel 60, Merck; Lösungsmittel-Gemisch: Chloroform:Methanol = 20:1).

Man erhält 21,7 g (53% der Theorie) 4-Cyclohexylphenyl-imidazol-1-yl-phenyl-methan vom Brechungsindex $n_D^{20} = 1{,}5831$.

*Herstellung der Ausgangsprodukte*

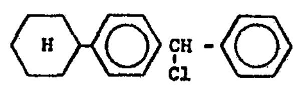

In ein Gemisch von 143 ml (2 Mol) Thionylchlorid und 200 ml Benzol werden bei 70°C 70 g (0,286 Mol) 4-Cyclohexylphenylphenylcarbinol portionsweise eingetragen. Das Reaktionsgemisch wird 15 Stunden auf 70°C erhitzt und anschließend eingedampft. Das verbleibende Oel wird mit Petroläther verrührt, wobei es zu einem Kristallbrei erstarrt. Man erhält 79 g (96% der Theorie) 4-Cyclohexylphenyl-phenylchlormethan vom Schmelzpunkt 75°C.

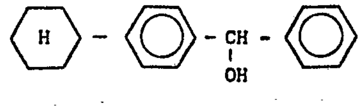

In eine Lösung von 92,5 g (0,35 Mol) 4-Cyclohexylbenzophenon in 300 ml Aethylalkohol werden portionsweise 6,4 g (0,17 Mol) Natriumboranat eingetragen. Das Reaktionsgemisch wird 15 Stunden unter Rückfluß erhitzt und dann auf Eis gegeben. Die organische Phase wird in Methylenchlorid aufgenommen, mit Wasser neutral gewaschen, über Natriumsulfat getrocknet, filtriert und eingedampft. Nach Verreiben mit Petroläther erhält man 77,3 g (83% der Theorie) 4-Cyclohexylphenyl-phenylcarbinol vom Schmelzpunkt 79°C.

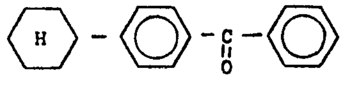

Ein Gemisch von 80 g (0,5 Mol) Cyclohexylbenzol, 70 g (0,5 Mol) Benzoylchlorid und 500 ml Methylenchlorid wird bei Raumtemperatur portionsweise mit 67 g (0,5 Mol) Aluminiumchlorid versetzt. Nach 15 Stunden wird das Reaktionsgemisch auf Eis/Salzsäure gegeben. Die organische Phase wird mit Wasser neutral gewaschen, über Natriumsulfat getrocknet und eingedampft. Das Rohprodukt wird destillativ gereinigt. Man erhält 93,3 g (75% der Theorie) 4-Cyclohexylbenzophenon vom Siedepunkt 160 bis $175°C/_{0,1}$ mm.

Beispiel 2

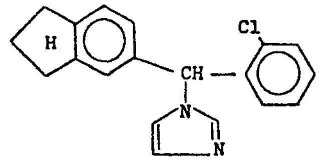

Zu einer Suspension von 85 g (1,25 Mol) Imidazol in 200 ml 1,2-Dichlorphenol werden bei 180°C 71 g (0,25 mol) 2-Chlorphenyl-indan-5-yl-chlormethan portionsweise eingetragen. Nach zweistündigem Rühren bei 180°C wird das Reaktionsgemisch am Rotationsverdampfer bei 80 bis 90°C eingedampft und der Rückstand in Methylenchlorid gelöst. Nach mehrmaligem Waschen der organischen Phase mit Wasser wird die Methylenchlorid-Lösung über Natriumsulfat getrocknet und eingedampft. Das verbleibende Oel wird chromatographisch gereinigt. (Absorbtionsmittel: Kieselgel 60, Merck; Lösungsmittel: Chloroform).

Man erhält 33,2 g (43% der Theorie) 2-Chlorphenyl-imidazol-1-yl-indan-5-yl-methan vom Brechungsindex $n_D^{20} = 1,6160$.

*Herstellung der Ausgangsprodukte*

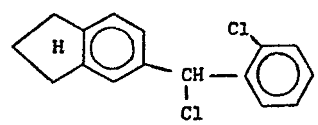

In ein Gemisch von 21,5 ml (0,26 Mol) Thionylchlorid und 150 ml Benzol werden bei 70°C 79,3 g (0.26 Mol) 2-Chlorphenyl-indan-5-yl-carbinol, gelöst in 150 ml Benzol, zugetropft. Nach fünfstündigem Erhitzen unter Rückfluß wird das Reaktionsgemisch eingedampft. Man erhält 71 g (98% der Theorie) 2-Chlorphenyl-indan-5-yl-chlormethan vom Brechungsindex $n_D^{20} = 1,6050$.

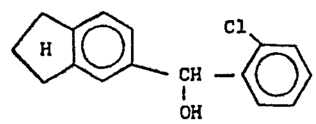

In eine Lösung von 79,6 g (0,3 Mol) 2-Chlorphenyl-indan-5-yl-keton in 500 ml Aethylalkohol werden portionsweise 5,7 g (0,15 Mol) Natriumboranat eingetragen. Das Reaktionsgemisch wird 4 Stunden auf 80°C erwärmt, dann auf Eis gegossen und mit Methylenchlorid extrahiert. Die organische Phase wird über Natriumsulfat getrocknet und eingedampft. Man erhält 79,3 g (98% der Theorie) 2-Chlorphenyl-indan-5-yl-carbinol vom Brechungsindex $n_D^{20} = 1,6012$.

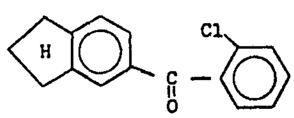

Ein Gemisch von 59 g (0,5 Mol) Indan, 87,5 g (0,5 Mol) 2-Chlorbenzoylchlorid und 200 ml Methylenchlorid wird bei Raumtemperatur portionsweise mit 66,7 g (0,5 Mol) Aluminiumchlorid versetzt. Nach Beendigung der Reaktion wird noch 4 Stunden nachgerührt und das Gemisch auf Eis/Salzsäure gegossen. Die organische Phase wird mit Wasser neutral gewaschen, über Natriumsulfat getrocknet und eingedampft. Das Rohprodukt wird durch Destillation gereinigt.

Man erhält 79,6 g (60% der Theorie) 2-Chlorphenyl-indan-5-yl-keton vom Siedepunkt $160°C/_{0,15}$ mm.

Beispiel 3

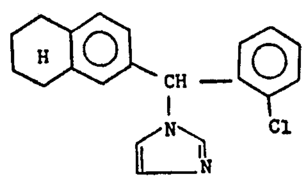

Zu einer Suspension von 51 g (0,75 Mol) Imidazol in 100 ml 1,2-Dichlorbenzol werden bei 180°C 44 g (0,15 Mol) 2-Chlorphenyl-1,2,3,4-Tetrahydronaphth-6-yl-chlormethan, gelöst in 50 ml 1,2-Dichlorbenzol, zugetropft. Das Reaktionsgemisch wird 2 Stunden auf 180°C erhitzt, nach dem Erkalten auf Wasser gegossen, die organische Phase mit Methylenchlorid verdünnt und mit Wasser gewaschen. Nach dem Trocknen über Natriumsulfat wird die Lösung im Vakuum eingedampft. Man erhält 26,8 g (55% der Theorie) 2-Chlorphenyl-imidazol-1-yl-1,2,3,4-tetrahydronaphth-6-yl-methan vom Brechungsindex $n_D^{20} = 1{,}6000$.

In entsprechender Weise werden die Verbindungen der nachfolgenden Tabelle 1 erhalten.

TABELLE 1

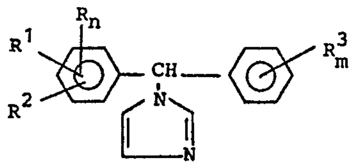

| Bsp Nr. | $R_n$ | $R^1$ | $R^2$ | $R^3m$ | Physik. Konstante |
|---|---|---|---|---|---|
| 4 | – | $3,4\text{-}(CH_2)_4$ | – | – | $n_D^{20}$ : 1.5912 |
| 5 | – | $3,4\text{-}(CH_2)_4$ | – | $2\text{-}CH_3$ | $n_D^{20}$ : 1,6040 |
| 6 | – | $3,4\text{-}(CH_2)_4$ | – | 4-Cl | $n_D^{20}$ : 1,5904 |
| 7 | – | $3,4\text{-}(CH_2)_3$ | – | – | $n_D^{20}$ : 1,5914 |
| 8 | – | $3,4\text{-}(CH_2)_3$ | – | $2\text{-}CH_3$ | $n_D^{20}$ : 1,6018 |
| 9 | – | $3,4\text{-}(CH_3)_3$ | – | 4-Cl | $n_D^{20}$ : 1,6031 |
| 10 | – | 4 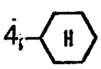 | H | 2-Cl | Fp: 104°C |
| 11 | – | $3,4\text{-}(CH_2)_4$ | – | 2-F | $n_D^{20}$ : 1,5890 |
| 12 | – | $3,4\text{-}(CH_4)_3$ | – | 2-F | $n_D^{20}$ : 1,6000 |
| 13 | – | 4 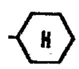 | H | 2-F | Fp: 102°C |
| 14 | – | 4 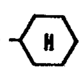 | H | 4-Cl | $n_D^{20}$ : 1,5867 |
| 15 | – | $3,4\text{-}(CH_2)_3$ | – | 2-F | Fp: 130°C (xHCl) |
| 16 | – | $3,4\text{-}(CH_2)_4$ | – | 2-F | Fp: 175°C (xHCl) |
| 17 | – | 4 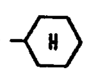 | H | 2-F | Fp: 175–176°C (xHCl) |
| 18 | – | 4 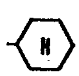 | H | $2,4\text{-}Cl_2$ | $n_D^{20}$ : 1,5848 |
| 19 | – | 4 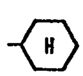 | H | $2,5\text{-}Cl_2$ | $n_D^{20}$ : 1,5795 |
| 20 | – | 4 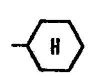 | H | 2-Br | Fp: 110°C |

**Patentansprüche**

1. Substituierte Diphenyl-imidazolyl-methane der allgemeinen Formel

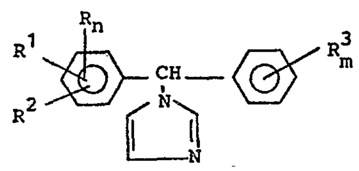

(I)

in welcher

R für Halogen, geradkettiges oder verzweigtes Alkyl oder Alkoxy mit jeweils 1 bis 4 Kohlenstoffatomen sowie für Halogenalkyl mit 1 bis 4 Kohlenstoffatomen und bis zu 3 Halogenatomen steht,

$R^1$ für gegebenenfalls einfach oder mehrfach durch Halogen oder Alkyl mit 1 bis 4 Kohlenstoffatomen substituiertes Cyclopalkyl mit 3 bis 7 Kohlenstoffatomen steht,

$R^2$ für Wasserstoff steht, oder

$R^1$ und $R^2$ gemeinsam in ortho-Stellung zueinander für eine gegebenenfalls einfach oder mehrfach durch Halogen oder Alkyl mit 1 bis 4 Kohlenstoffatomen substituierte Methylenbrücke mit 3 bis 5 Methylengruppen stehen,

$R^3$ für Halogen, geradkettiges oder verzweigtes Alkyl mit 1 bis 4 Kohlenstoffatomen, Halogenalkyl mit 1 bis 2 Kohlenstoffatomen und bis zu 3 Halogenatomen, Alkoxy, Alkylthio und Alkylsulfonyl mit jeweils 1 bis 2 Kohlenstoffatomen, Amino, Nitro oder Cyano, und

m und n für ganze Zahlen von 0 bis 3 stehen,

und deren physiologisch verträglichen Säureadditionssalze.

2. Verfahren zur Herstellung von Diphenyl-imidazolyl-methanen der allgemeinen Formel (I), dadurch gekennzeichnet, daß man Diphenyl-halogen-methane der allgemeinen Formel

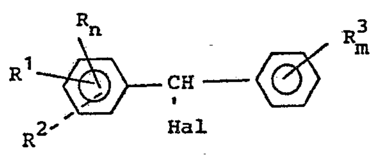

(II)

in welcher

R, $R^1$, $R^2$, $R^3$, m und n die oben angegebene Bedeutung haben und

Hal für Halogen steht,

mit Imidazol, gegebenenfalls in Gegenwart eines Säurebindemittels und gegebenenfalls in Gegenwart eines Verdünnungsmittels, umsetzt.

3. Arzneimittel, gekennzeichnet durch einen Gehalt an mindestens einem Diphenyl-imidazolyl-methan der allgemeinen Formel (I) und üblichen Hilfs- und Trägerstoffen.

**Revendications**

1. Des diphényl-imidazolyl-méthanes substitués de formule générale (I):

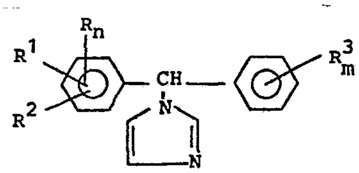

(I)

dans laquelle:

R désigne un halogène, un groupe alkyle ou un groupe alkoxy à chaîne droite ou ramifiée ayant chacun 1 à 4 atomes de carbone ainsi qu'un groupe halogènalkyle ayant 1 ou 2 atomes de carbone et jusqu'à 3 atomes d'halogènes,

$R^1$ est un groupe cycloalkyle ayant 3 à 7 atomes de carbone, éventuellement substitué une ou plusieurs fois par un halogène ou un radical alkyle ayant 1 à 4 atomes de carbone,

$R^2$ est de l'hydrogène, ou bien

$R^1$ et $R^2$ forment ensemble en position ortho l'un par rapport à l'autre un pont méthylénique de 3 à 5 groupes méthylène éventuellement substitué une ou plusieurs fois par un halogène ou un radical alkyle ayant 1 à 4 atomes de carbone,

$R^3$ est un halogène, un groupe alkyle à chaîne droite ou ramifiée ayant 1 à 4 atomes de carbone, un groupe halogénalkyle ayant 1 ou 2 atomes de carbone et jusqu'à 3 atomes d'halogènes, un groupe alkoxy, un groupe alkylthio et un groupe alkylsulfonyle ayant chacun 1 ou 2 atomes de carbone, un groupe amino, un groupe nitro ou un groupe cyano, et

*m* et *n* sont des nombres entiers de 0 à 3,

et leurs sels d'addition d'acides acceptables du point de vue physiologique.

2. Procédé de production de diphényl-imidazolyl-méthanes de formule générale (I), caractérisé en ce qu'on fait réagir des diphényl-halogéno-méthanes de formule générale (II):

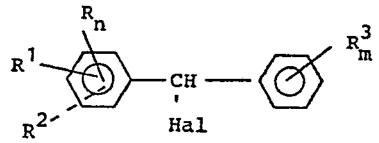

(II)

dans laquelle:

R, $R^1$, $R^2$, $R^3$, *m* et *n* ont la définition indiquée ci-dessus et

Hal est un halogène,

avec l'imidazole, le case échéant en présence d'un accepteur d'acide et en la présence éventuelle d'un diluant.

3. Médicament, caractérisé par une teneur en au moins un diphényl-imidazolyl-méthane de formule générale (I) et en substances auxiliaires et supports classiques.

## Claims

1. Substituted diphenyl-imidazolyl-methanes of the general formula (I)

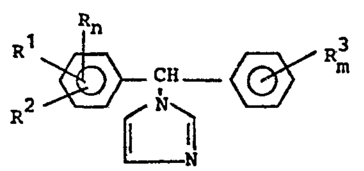

(I)

in which

R represents halogen, straight-chain or branched alkyl or alkoxy with in each case 1 to 4 carbon atoms, and halogenoalkyl with 1 to 2 carbon atoms and up to 3 halogen atoms,

$R^1$ represents cycloalkyl with 3 to 7 carbon atoms which is optionally monosubstituted or polysubstituted, the substituents being halogen or alkyl with 1 to 4 carbon atoms,

$R^2$ represents hydrogen, or

$R^1$ and $R^2$, in the ortho position relative to one another, together represent a methylene bridge with 3 to 5 methylene groups which is optionally monosubstituted or polysubstituted, the substituents being halogen or alkyl with 1 to 4 carbon atoms,

$R^3$ represents halogen, straight-chain or branched alkyl with 1 to 4 carbon atoms, halogenoalkyl with 1 to 2 carbon atoms and up to 3 halogen atoms, alkoxy, alkylthio and alkylsulphonyl with in each case 1 to 2 carbon atoms, amino, nitro or cyano and

m and n represent integers from 0 to 3,

and their physiologically acceptable acid addition salts.

2. Process for the production of diphenyl-imidazolyl-methanes of the general formula (I), characterised in that diphenyl-halogeno-methanes of the general formula (II)

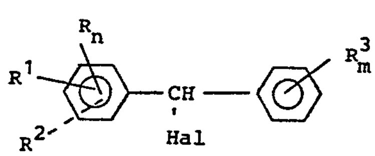

(II)

in which

R, $R^1$, $R^2$, $R^3$, m and n have the above-mentioned meaning and

Hal represents halogen,

are reacted with imidazole, optionally in the presence of an acid-binding agent and optionally in the presence of a diluent.

3. Medicaments, characterised in that they contain at least one diphenyl-imidazolyl-methane of the general formula (I) and customary auxiliaries and carriers.